# EUROPEAN PATENT APPLICATION

(11) **EP 4 039 188 A1**
(43) Date of publication of application: **10.08.2022**
(21) Application number: 19952040.4
(22) Date of filing: 11.11.2019
(51) Int. Cl.: A61B 5/107, A61B 5/00

(54) **BODY TYPE ANALYSIS DEVICE**

(30) Priority: 08.11.2019 KR 20190142750
(71) Applicant: CBH Ltd., Jeonju-si, Jeollabuk-do 54849 (KR)
(72) Inventor: YOON, Jong Kyu, Gwangyang-si Jeollanam-do 57789 (KR)
(74) Representative: BCKIP
(86) International application number: PCT/KR2019/015275
(87) International publication number: WO 2021/090991

(57) **Abstract**

The present disclosure relates to a body type analysis device and, particularly, to a body type analysis device comprising: a detection unit which is provided to be movable in the vertical direction on the back of a person undergoing detection, and which detects the vertebral line of the person undergoing detection; and a tracking unit for moving the detection unit in the straight vertical direction, wherein the detection unit comprises a center detection part for performing measurement in the range of the normal vertebral line, and at least one side detection part provided on each of the left and right sides of the center detection part so as to perform measurement in the range of the abnormal vertebral line, and the center detection part and the side detection part are provided to detect whether the spine is bent through the rotational angle of rotating in the anterior-posterior direction of the spine, and thus the present disclosure provides the advantages of reducing body type analysis time and enabling more accurate data to be ensured.

## Description

### [Technical Field]

The present disclosure relates to a body type analysis device capable of accurately analyzing a shape of a spine.

### [Background Art]

A spine refers to a bone that supports the main skeletons of a person including neck, back, waist, hip, and tail portions. The skeleton of the person is classified into 7 cervical vertebrae, 12 thoracic vertebrae, 5 lumbar vertebrae, 5 sacra, and 4 coccyges. In the spine, there is a spinal cord as a bundle of nerves output from a brain, which serves to connect the brain, which is a central nervous system, and peripheral organs, which is a peripheral nervous system. The spinal cord is a very important neural passage, and is protected by the spine, which is a strong bone, because various types of paralysis are possible when the spinal cord is damaged.

Since spinal diseases are often caused by wrong lifestyle for a long time or occupational activities, even those who normally thought that there is no problem with the spine may unconsciously have spinal problems over time.

Since the spine serves as a neural passage as described above, and supports the skeleton of the human body, various diseases may be caused when the spine deviates from its normal shape, and thus it is very important to maintain the shape of the spine.

In many cases, since people should sit in a chair for a long time, various types of functional chairs for spine protection are released to protect the spine, and expensive chairs equipped with a spine treatment function are also being released.

Spinal diseases may have at least five or six different diseases even as representative diseases, and each disease is subdivided depending on the deformation of the shape of the spine for each disease. Accordingly, the functional chairs released for spinal treatment only focus on bringing the spine closer to the normal shape of the spine, and thus it is difficult to prepare for various types of spinal diseases. In particular, chairs equipped with a spinal disease correction function have only one form of operation, and thus may have no effect at all or even have a very harmful effect depending on the spinal disease.

Accordingly, the first thing to be done is to properly identify the shape of each individual's spine. However, X-rays or other CT scans, which are the easiest methods to identify the shape of an individual's spine, are not preferable because the human body is exposed to considerable radiation even in a single scan.

The shape of the spine by methods other than radiography is usually diagnosed by an expert groping and tracking the spine from top to bottom using an instrument. Since the shape of the spine and the types of diseases are so diverse, in this process, the person who is not a highly skilled expert may not properly follow the shape of the spine, thereby obtaining an incorrect shape of the spine different from the real shape.

Due to such a problem, it is inevitably difficult to manufacture an instrument for diagnosing spinal diseases while simply following the shape of the spine. This is because the curvature of the spine exposed to the outside of the human body is not very clear, and thus the sensor deviates from the process of following the trajectory of the spine even when the sensor is sensitively and freely changeable.

### [Summary of Invention]

### [Technical Problem]

The present disclosure has been made in efforts to solve the above technical problems, and an object of the present disclosure is to provide a body type analysis device, which may accurately diagnose the real types of spinal diseases even without the help of an expert, and accurately analyze the shape of the spine without errors even in a state in which the person wears clothes.

The technical objects of the present disclosure are not limited to the above-mentioned technical objects, and other objects not mentioned may be clearly understood by those skilled in the art from the following description.

### [Solution to Problem]

In order to achieve the object, there is provided a body type analysis device according to one embodiment of the present disclosure including: a detection unit provided to be vertically movable on a back portion of a person to be detected, and configured to detect a shape of a spine of the person to be detected based on the amount of rotation rotated by interfering with a curve of the spine of the person to be detected and a moving drive unit configured to move the detection unit in a straight vertical direction on the back portion of the person to be detected.

Here, the detection unit may include a center detection part configured to measure the shape of the spine in a scope of a left-right width of a normal spine line and a pair of side detection parts provided to be spaced in the left and right of the center detection part by a predetermined distance, respectively, and configured to measure the shape of the spine in a scope of a left-right width of an abnormal spine line.

In addition, the center detection part and the side detection part may be provided to be inclined to form an obtuse angle with a moving direction of the detection unit, and may calculate the shape of the spine through a combination of the respective amounts of rotation of the center detection part and the side detection part.

In addition, the detection unit further may include a center encoder configured to detect a rotation angle of the center detection part and a pair of side encoders configured to detect rotation angles of the pair of side detection parts.

In addition, the center detection part and the pair of side detection parts may be provided to be elastically rotatable around an arbitrary rotation shaft.

In addition, the center encoder and the pair of side encoders may be installed on the arbitrary rotation shaft.

In addition, the detection unit may be configured so that a separation distance between the pair of side detection parts is set to a distance capable of detecting a range of the shape of the spine diagnosed as a scoliosis patient with only one-time operation of the moving drive unit.

In addition, the moving drive unit may include a moving bogie part configured to provide an installation location of the detection unit, a moving rail part configured to provide a moving line to move the moving bogie part in a straight vertical direction, and a moving drive part configured to provide a driving force to move the moving bogie part along the moving rail part.

In addition, the moving rail part may include a screw rod arranged along the moving line, and having a male thread processed on an outer circumferential surface to be moved by an operation rotated by passing through a moving block provided on the moving bogie part and a rod support bracket configured to rotationally support both upper and lower ends of the screw rod.

In addition, any one of the upper and lower ends of the screw rod may be directly connected to a rotation shaft of the moving drive part.

In addition, the moving rail part may further include a sub rail provided to be spaced apart from the screw rod by a predetermined interval and parallel to the screw rod, and configured to support the moving of the moving bogie part.

In addition, the moving drive unit may be installed to be movable with respect to an analysis body part fixed to be vertically long.

In addition, the analysis body part may be provided with a fabric guide belt to be connected to the upper and lower ends of the moving drive unit and rotated in conjunction therewith.

In addition, one side of the analysis body part may be provided with a protective chain rail configured to protect an electric wire arranged to supply power to the moving drive unit, and an electric wire protective chain moved to cover the electric wire arranged on the protect chain rail may be coupled to the moving drive unit to be interlocked with the moving drive unit.

### [Advantageous Effects of Invention]

The body type analysis device according to one embodiment of the present disclosure can achieve various effects as follows.

First, it is possible to detect the shape of the spine of the person to be detected with only one-time detection process, thereby removing the inconvenience of the person to be detected who has difficulty in moving.

Second, it is possible to estimate the shape of the spine based on the total and continuous rotation amount of the center detection part and the pair of side detection parts and the change thereof, thereby improving accuracy, and improving reliability of the product.

### [Brief Description of Drawings]

FIG. 1 is a perspective view showing a body type analysis device according to one embodiment of the present disclosure.
FIG. 2 is a perspective view from another direction of FIG. 1 and a partial enlarged view.
FIG. 3 is a side view of FIG. 1.
FIG. 4 is a bottom view of FIG. 1.
FIG. 5 is a perspective view showing a moving drive unit and a detection unit in a configuration of FIG. 1.
FIG. 6 is a side view of FIG. 4, and a side view showing an operation process of the detection unit.
FIG. 7 is a front view of FIG. 4.
FIGS. 8A and 8B are a rear view and a side view showing a measurement process using the body type analysis device according to one embodiment of the present disclosure.

### [Description of Embodiments]

Hereinafter, some embodiments of the present disclosure will be described in detail with reference to exemplary drawings. In adding reference numerals to the components of each drawing, it should be noted that the same components are given the same reference numerals as much as possible even when they are indicated in different drawings. In addition, in describing the embodiment of the present disclosure, when it is determined that a detailed description of a related known configuration or function interferes with the understanding of the embodiment of the present disclosure, the detailed description thereof will be omitted.

In describing the components according to the embodiment of the present disclosure, terms such as first, second, A, B, (a), (b), etc. may be used. These terms are only for distinguishing the components from another, and the essence, order, or sequence of the corresponding components are not limited by the terms. In addition, unless otherwise defined, all terms used herein, including technical or scientific terms, have the same meaning as commonly understood by those skilled in the art to which the present disclosure pertains. Terms such as those defined in a commonly used dictionary should be interpreted as having a meaning consistent with the meaning in the context of the related art, and should not be interpreted in an ideal or excessively formal meaning unless explicitly defined in the present application.

Throughout the specification, when a certain portion 'includes' or 'has' a certain component, it means that other components may be further included, other than precluding other components, unless otherwise stated.

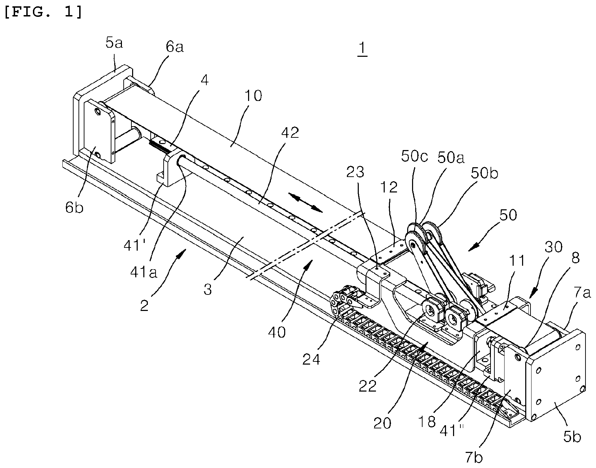

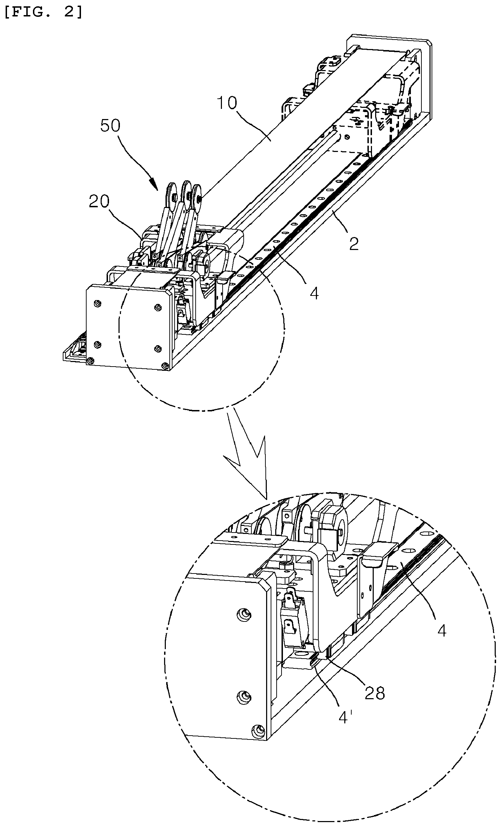

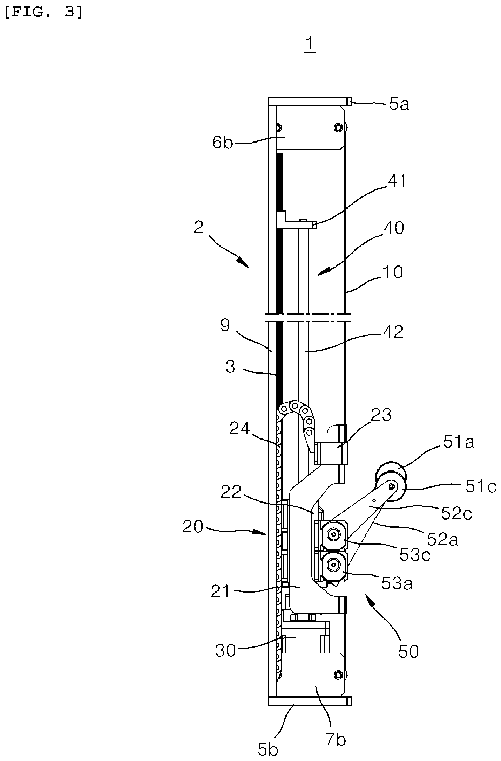

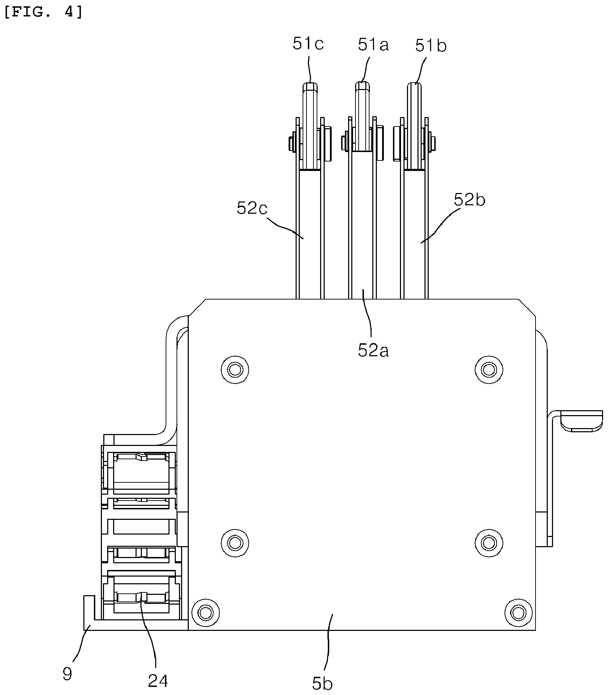

FIG. 1 is a perspective view showing a body type analysis device according to one embodiment of the present disclosure, FIG. 2 is a perspective view from another direction of FIG. 1 and a partial enlarged view, FIG. 3 is a side view of FIG. 1, and FIG. 4 is a bottom view of FIG. 1.

As shown in FIG. 1, a body shape analysis device 1 according to one embodiment of the present disclosure includes a detection unit 50 provided to be vertically movable at a back portion of a person to be detected with a fixed posture, and configured to detect the shape of a spine of the person to be detected based on the amount of rotation rotated by interfering with the curvature of a spine of the person to be detected, and a moving drive unit 20 configured to move the detection unit 50 at a back portion of the person to be detected in a straight vertical direction.

Here, the person to be detected may be defined as a person who is expected to have a spinal disease such as scoliosis or a person who uses the body shape analysis device 1 according to the present disclosure in order to diagnose the spinal disease.

The body shape analysis device 1 according to one embodiment of the present disclosure may serve to provide data capable of detecting and analyzing the shape of the spine as a pre-step for medical treatment of the person to be detected. However, the body shape analysis device 1 according to one embodiment of the present disclosure is not necessarily limited to the above-described analysis of the spine portion, and may be used for any purpose as long as it may detect the external shape of the body, which will also belong to the scope of the present disclosure.

Although not shown in the drawing, the detection unit 50 and the moving drive unit 20 may be coupled to a height-adjustable chair (not shown) on which the person to be detected may be seated via an analysis body part 2. The height-adjustable chair may be an auxiliary instrument for allowing the shape of the spine with a stable fixed posture to be detected using the body shape analysis device 1 according to one embodiment of the present disclosure regardless of the age and body type of the person. However, the detection unit 50 and the moving drive unit 20 do not necessarily have to be mounted on the height-adjustable chair, and may be mounted on any device capable of maintaining the person to be detected in a stable fixed posture.

As shown in FIG. 1, the analysis body part 2 may include a body plate 3 formed to be vertically long, and mounting panels 5a, 5b provided at upper and lower ends of the body plate 3, respectively to serve to mediate the coupling to the height-adjustable chair. A sub rail 4 configured to assist the vertical moving of a moving bogie part (not shown) of the moving drive unit 20 to be described later may be disposed on any one of the left end and the right end of the analysis body part 2, and a protective chain rail 9 provided to protect electric wires for applying power to the moving drive unit to be described later may be disposed the other one of the left end and the right end of the analysis body part 2. The sub rail 4 and the protective chain rail 9 will be described in more detail later.

The mounting panels 5a, 5b have the shape with an opened one side, and may be connected to a pair of mounting plates 6a, 6b, 7a, 7b having a vertical cross section of about "Π" shape. The mounting plates 6a, 6b of the upper mounting panel 5a provided at the upper end of the body plate 3 may be disposed so that the opened portion is directed in a downward direction, and the mounting plates 7a, 7b of the lower mounting panel 5b provided at the lower end of the body plate 3 may be disposed so that the opened portion is directed in an upward direction.

Each of a pair of mounting plates 6a, 6b, 7a, 7b each may be provided with a belt support bar 8 configured to rotationally support a fabric guide belt 10 to be described later. The belt support bar 8 may be connected while laterally traversing opposite spaces formed in the mounting plates 6a, 7a provided at one side and the mounting plates 6b, 7b provided at the other side.

Meanwhile, the analysis body part 2 may be provided with the fabric guide belt 10 to be connected to the upper and lower ends of the moving drive unit 20 to be described later to be rotated in conjunction therewith. The fabric guide belt 10 may be made of fabric, one end 12 of which may be connected to the upper end of the moving drive unit 20, and the other end 11 of which may be connected to the lower end of the moving drive unit 20.

In addition, the fabric guide belt 10 surrounds the belt support rod 8 provided to transverse the mounting plates 6a, 6b of the above-described upper mounting panel 5a and is concealed toward a rear surface of the body play 3 of the analysis body part 2 in a state of having one end 12 connected to the upper end of the moving drive unit 20, and is exposed from the body plate 3 to extend from the rear side of the body plate 3 to surround the mounting plates 7a, 7b of the lower mounting panel 5b and has the other end 11 connected to the lower end of the moving drive unit 20. Accordingly, the fabric guide belt 10 may be rotationally moved at the front and rear sides of the body plate 3 as a whole according to the movement of the moving drive unit 20.

Here, the fabric guide belt 10 is made of a fabric material, and when the detection unit 50 to be described later detects the shape of the spine for the back portion of the person to be detected, the fabric guide belt 10 comes into close contact with the back portion of the person to be detected, thereby preventing the person to be detected from feeling a sense of heterogeneity.

Meanwhile, the detection unit 50 may detect a protruding portion of the spine of the person to be detected by an operation of being vertically moved by the operation of the moving drive unit 20 at the back portion of the person to be detected to track the shape of the spine. In other words, the detection unit 50 may detect the shape of the spine by tracking from the upper end of the spine to the lower end of the spine while maintaining a state of coming into direct contact with the protruding portion of the spine of the person to be detected.

The moving drive unit 20 may move downward the detection unit 50 from the upper side to the lower side of the analysis body part or may move upward the detection unit 50 from the lower side to the upper side of the analysis body part.

More specifically, the moving drive unit 20 may include a moving bogie part that provides an installation location of the detection unit 50, a moving rail part 40 that provides a moving line to move the moving bogie part in a straight vertical direction, and a moving drive part 30 that provides a driving force to move the moving bogie part along the moving rail part 40.

As shown in FIG. 1, the moving bogie part may include a moving frame 21 forming a skeleton, and a bogie mounting plate 22 coupled to the inside of the moving frame 21 to be parallel to the moving direction.

The moving frame 21 is provided as a frame of a quadrangular shape with an approximately center open in a front-rear direction, and a portion to which the bogie mounting plate 22 is coupled may have a shape that is recessed in a further rearward direction of the back portion of the person to be detected.

The bogie mounting plate 22 may be coupled to the portion recessed to the rear of the moving frame 21 to be parallel to the moving direction, and the detection unit 50 may be coupled to a front surface of the bogie mounting plate 22.

Meanwhile, the moving rail part 40 may include a screw rod 42 arranged along the moving line to be vertically long to be spaced apart from the front surface of the analysis body part 2 formed to be vertically long by a predetermined distance and parallel thereto, and having the shape of a male thread processed on an outer circumferential surface to be moved by an operation rotated by passing through a moving block 18 provided on the moving bogie part, and a rod support bracket 41 configured to rotationally support both upper and lower ends of the screw rod 42.

In other words, the male thread may be processed on the outer circumferential surface of the screw rod 42, and the portion with the male thread processed may be fastened by passing through a screw hole (reference numeral not indicated) in which a female thread of the moving block 18 is formed.

The moving block 18 may serve as a medium that vertically moves the moving frame 21 as it is fixed to a lower end of the moving frame 21 of the moving bogie part, and vertically moved by the mutual engagement between the male thread of the screw rod 42 and the female thread of the moving block 18 as described above while the screw rod 42 is axially rotated in place by the moving drive part 30 to be described later.

The rod support bracket 41 may be formed to protrude forward from a front upper side and a front lower side of the analysis body part 2 by a predetermined length so that upper and lower ends of the screw rod 42 are rotationally supported by passing through the rod support bracket 41. An upper rod support bracket 41' formed at the front upper side of the analysis body part 2 and a lower rod support bracket 41" formed at the front lower side of the analysis body part 2 may be formed with a support hole 41a through which the upper rod support bracket 41' and the lower rod support bracket 41" vertically pass, respectively, and although not shown in the drawing, a rotation support bearing configured to rotationally support the outer circumferential surface of the screw rod 42 may be embedded in the support hole 41a.

Meanwhile, the moving drive part 30 may be provided as an electric motor electrically driven to move the moving bogie part. Any one of the upper and lower ends of the screw rod 42 may be directly connected to a rotation shaft of the moving drive part 30 provided as the electric motor. Here, the moving drive part 30 may be provided as a BLDC motor in which noise generation is minimized to provide a sense of stability to the person to be detected.

The moving rail part 40 may further include the sub rail 4 spaced apart from the screw rod 42 by a predetermined distance and parallel thereto, and configured to support the moving of the moving bogie part. The sub rail 4 may be disposed on any one of the left and right ends of the analysis body part 2 to be vertically long.

More specifically, as shown in FIG. 2, the sub rail 4 is provided with a rail groove 4' grooved to be vertically long on a side surface, and serves to guide the sliding movement of the moving frame 21 when the moving bogie part moves by inserting a part of the rail block 28 provided under the moving frame 21 of the moving bogie part into the rail groove 4', and at the same time, prevent the moving frame 21 from moving and swinging laterally.

Meanwhile, the other of the left and right ends of the analysis body part 2 may be further provided with a protective chain rail 9 disposed to be vertically long. Although not shown in the drawing, the protective chain rail 9 is to cover an electric wire provided to apply power to the moving drive part 30, and provides a path that allows an electric wire protective chain 24 connected to and interlocked with one side of the moving frame 21 of the moving bogie part to be slidably moved.

More specifically, although not shown in the drawing, as described above, the moving drive part 30 may be provided as the electric motor electrically driven, and the electric wire may be arranged to supply the power. The electric wire is arranged on the protective chain rail 9, and the electric wire protective chain 24 connected to the moving frame 21 via the chain bracket 23 serves to protect the electric wire arranged on the protective chain rail 9 while being slidably moved in conjunction with the moving bogie part in order to prevent the electric wires arranged on the protective chain rail 9 from interfering with each other when the moving bogie part moves.

When the power is applied to the moving drive unit 30 provided as the electric motor and the rotation shaft is rotationally operated, the moving drive unit 20 is rotated in place as the screw rod 42 directly connected to the rotation shaft of the moving drive part 30 receives the rotation support of the rod support bracket 41, the moving block 18 having the screw hole formed with the female thread engaged with the male thread of the screw rod 42 is moved along the screw rod 42 in an upward or downward direction of the analysis body part 2 according to the rotation direction of the screw rod 42, so that the detection unit 50 coupled to the moving bogie part may be vertically moved. At this time, since the moving bogie part is moved while guided by the screw rod 42 and the sub rail 4, the moving bogie part may be stably slidably moved without swinging laterally.

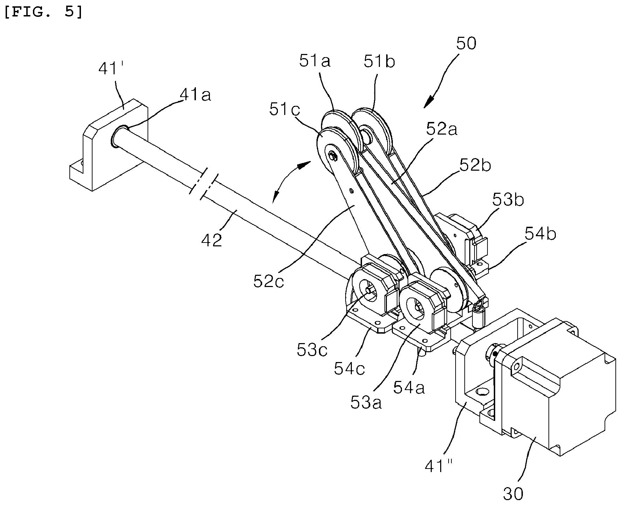

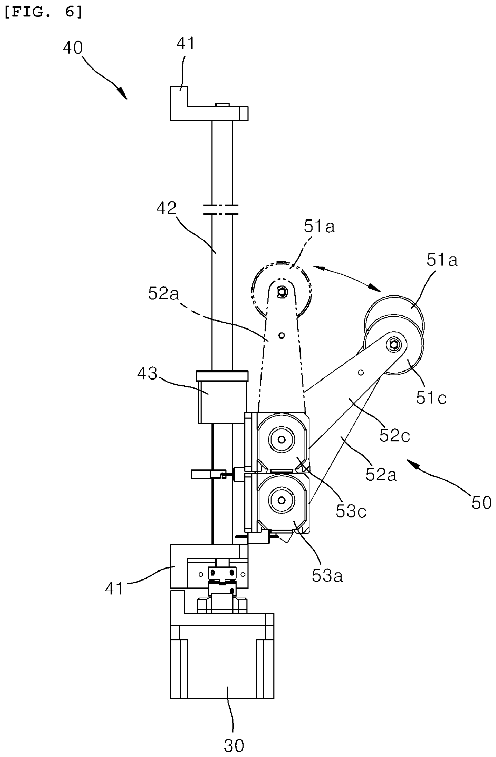

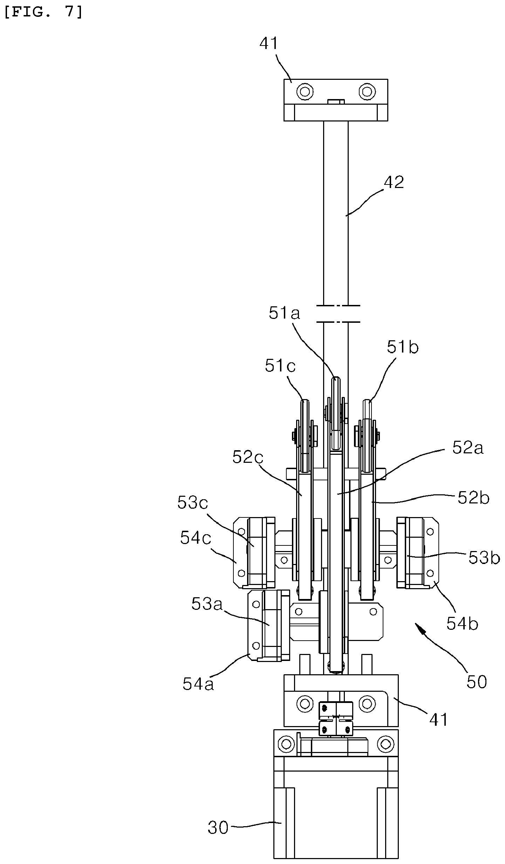

FIG. 5 is a perspective view showing a moving drive unit and a detection unit in a configuration of FIG. 1, FIG. 6 is a side view of FIG. 4, and a side view showing an operation process of the detection unit, and FIG. 7 is a front view of FIG. 4.

As shown in FIGS. 5 to 7, the detection unit 50 may be coupled to the moving drive unit 20 via the bogie mounting plate 21 in the configuration of the moving bogie part.

More specifically, as described above, the bogie mounting plate has a configuration that is vertically disposed at the portion recessed in a groove shape in a backward direction of the moving frame 21 to provide the installation location of the detection unit 50, and the detection unit 50 may be coupled to the front surface of the bogie mounting plate 21.

As shown in FIG. 5, the detection unit 50 may include a center detection part 50a configured to measure the shape of the spine in the scope of the left-right width of the normal spine line, and a pair of side detection parts 50b, 50c provided to be spaced by a predetermined distance in the left and right of the center detection part 50a, and configured to measure the shape of the spine in the scope of the left-right width of the abnormal spine line.

Here, the scope of the left-right width of the normal spine line refers to the scope of the average spine line of the normal person who does not suffer from spinal disease such as scoliosis, and the scope of the left-right width of the abnormal spine line is a concept including deviating from the scope of the left-right width of the normal spine line.

The center detection part 50a and the pair of side detection parts 50b, 50c may be provided to be inclined to form an obtuse angle with the moving direction of the detection unit 50.

For example, as shown in FIG. 4, when the upper left portion of the drawing is an upper side of the body type analysis device 1 according to one embodiment of the present disclosure, the moving bogie part may be moved from the upper left portion in the drawing (actually, upper side of the back portion of the person to be detected) to the lower right portion in the drawing (actually, lower side of the back portion of the person to be detected) in order to detect the shape of the spine of the person to be detected, and the center detection part 50a and the pair of side detection parts 50b, 50c may be disposed to be inclined to form an obtuse angle with the moving direction of the detection unit 50.

Here, the center detection part 50a and the pair of side detection parts 50b, 50c may include a center rotation link 52c and the side rotation links 54b, 54c rotatably connected to the front surface of the bogie mounting plate 21 via the link connection brackets 54a, 54b, 54c, and a center roller 51a and side rollers 51b, 51c rotatably provided at the tip of the center rotation link 52c, and the respective tips of the side rotation links 54b, 54c.

The link connection brackets 54a, 54b, 54c may mediate the rotation coupling of the center rotation link 52c, and the rotation coupling of the pair of side rotation links 54b, 54c, respectively, and mediate the coupling between a center encoder 53a and a pair of side encoders 53b, 53c, which will be described later.

Here, the center detection part 50a and the pair of side detection parts 50b, 50c may be provided to be elastically rotatable around an arbitrary rotation shaft. The arbitrary rotation shaft may be a hinge shaft connected to each of the link connection brackets 54a, 54b, 54c, and a hinge spring interposed in the hinge shaft (not shown) so that the center rotation link 52c and the side rotation link 54b, 54c may be elastically rotated, respectively. However, an elastic member which enables the elastic rotation of the center rotation link 52c and the side rotation links 54b, 54c is not necessarily limited to the hinge spring, and may also adopt any configuration as long as it is a configuration that may elastically rotate them so that the tips of the center rotation link 52c and the side rotation links 54b, 54c come into close contact with the back portion of the person to be detected.

Meanwhile, the center roller 51a and the side rollers 51b, 51c may be provided in the form of a rotating wheel as portions coming into direct contact with the back portion of the person to be detected, thereby minimizing the pain that the person to be detected feels while the detection of the shape of the spine is demonstrated through the body type analysis device 1 according to one embodiment of the present disclosure.

As shown in FIG. 5, the body shape analysis device 1 according to one embodiment of the present disclosure may calculate the shape of the spine through the combination of the amount of rotation rotated as the center detection part 50a and the side detection parts 50b, 50c come into contact with the back portion of the person to be detected who is a measurement object.

To this end, the detection unit 50 may further include the center encoder 53a configured to detect a rotation angle of the center detection part 50a, and the pair of side encoders 53b, 53c configured to detect rotation angles of the pair of side detection units 50b, 50c.

The center encoder 53a and the side encoders 53b, 53c are connected to an arbitrary rotation shaft connecting the center rotation link 52a and the pair of side rotation links 52b, 52c to the link connection brackets 54a, 54b, 54c, respectively to serve to measure the amount of rotation of each of the center rotation link 52a and the pair of side rotation links 52b, 52c that are rotated by interfering with the back portion of the person to be detected.

In other words, the center encoder 53a and the side encoders 53b, 53c may detect the amount of rotation of the center rotation link 52a and the pair of side rotation links 52b, 52c simultaneously and continuously when the detection unit 50 is moved only once, so that it is possible to estimate the shape of the spine of the person to be detected.

In order to enable the shape of the spine of the person to be detected to be completely detected by only one-time moving operation of the detection unit 50, the detection unit 50 is preferably configured so that a separation distance between the pair of side detection parts 50b, 50c is set to a distance at which the range of the shape of the spine diagnosed as a scoliosis patient may be at least detected by only one-time operation of the moving drive unit 20.

The separation distance between the pair of side detection parts 50b, 50c is preferably set by collecting a vast amount of information on the shape of the spine diagnosed as a patient with a spinal disease (e.g., scoliosis) in the medical industry.

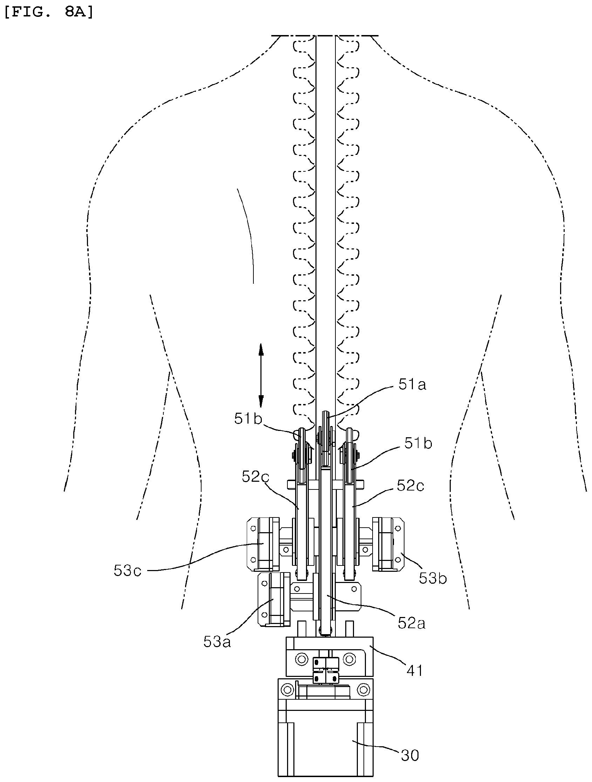

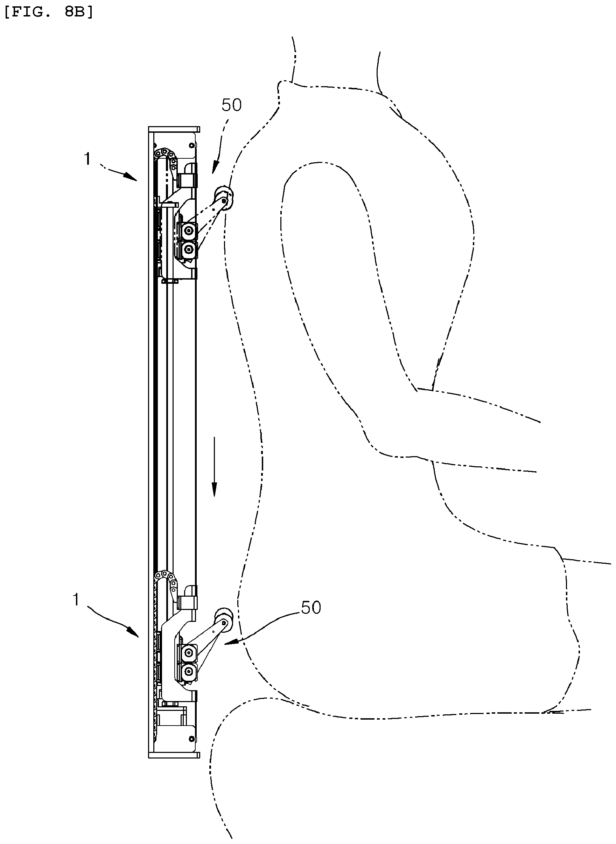

FIGS. 8A and 8B are a rear view and a side view showing a measurement process using the body type analysis device according to one embodiment of the present disclosure.

A process of detecting the shape of the spine of the person to be detected using the body shape analysis device 1 according to one embodiment of the present disclosure configured as described above will be briefly described with reference to FIGS. 8A and 8B.

First, as shown in FIGS. 8A and 8B, after allowing the person to be detected to be seated in the height-adjustable chair on which the body shape analysis device 1 according to one embodiment of the present disclosure is installed to maintain the fixed posture, or to stand to maintain the fixed posture, the body type analysis device 1 is operated by applying power to the moving drive unit 20.

At this time, it is preferable that an initial location of the detection unit 50 is relatively located at the upper side of the back portion of the person to be detected, and the shape of the spine of the person to be detected may be completely detected as the moving bogie part of the moving drive unit 20 is moved from the upper portion to the lower portion of the person to be detected only once.

Here, the center detection part 50a and the pair of side detection parts 50b, 50c may form an obtuse angle with the moving direction (i.e., in a downward direction) to be easily pushed in a backward direction or elastically rotated in a forward direction by a curve according to the back portion (or the shape of the spine) of the person to be detected.

When the center detection part 50a has a change in the predetermined amount of rotation while measuring the shape of the spine in the scope of the left-right width of the normal spine line, the pair of side detection parts 50b, 50c also have a slight change in the amount of rotation while measuring the shape of the spine in the scope of the left-right width of the abnormal spine line. Here, the respective amounts of rotation detected by the center encoder 53a and the pair of side encoders 53b, 53c are measured as different values, respectively, and the shape of the spine of the person to be detected may be calculated by combining data of the above amount of rotation as a whole while the only one-time moving operation of the moving drive unit 20.

In detecting the shape of the spine of the patient (person to be detected) who suffers from the spinal disease (e.g., scoliosis), the body shape analysis device 1 according to one embodiment of the present disclosure may detect the complete shape of the spine only with the one-time detection process, thereby removing the inconvenience of the patient (person to be detected) with the spinal disease who has difficulty in moving and are passive in the detection process, and also accurately detect the shape of the spine, thereby greatly improving the reliability of the product.

The above-described present disclosure is not limited by the aforementioned embodiment and the accompanying drawings, and it will be apparent to those skilled in the art to which the present disclosure pertains that various substitutions, modifications, and changes are possible without departing from the technical spirit of the present disclosure.

### [Industrial Applicability]

The present disclosure provides the body type analysis device including a detection unit provided to be vertically movable on the back portion of the person to be detected, and configured to detect the shape of the spine of the person to be detected based on the amount of rotation rotated by interfering with the curve of the spine of the person to be detected, and a moving drive unit configured to move the detection unit in the straight vertical direction on the back portion of the person to be detected.

## Claims

1. A body type analysis device comprising:
a detection unit provided to be vertically movable on a back portion of a person to be detected, and configured to detect a shape of a spine of the person to be detected based on the amount of rotation rotated by interfering with a curve of the spine of the person to be detected; and
a moving drive unit configured to move the detection unit in a straight vertical direction on the back portion of the person to be detected.

2. The body type analysis device of claim 1,
wherein the detection unit includes:
a center detection part configured to measure the shape of the spine in a scope of a left-right width of a normal spine line; and
a pair of side detection parts provided to be spaced in the left and right of the center detection part by a predetermined distance, respectively, and configured to measure the shape of the spine in a scope of a left-right width of an abnormal spine line.

3. The body type analysis device of claim 2,
wherein the center detection part and the side detection part are provided to be inclined to form an obtuse angle with a moving direction of the detection unit, and
calculate the shape of the spine through a combination of the respective amounts of rotation of the center detection part and the side detection part.

4. The body type analysis device of claim 2,
wherein the detection unit further includes:
a center encoder configured to detect a rotation angle of the center detection part; and
a pair of side encoders configured to detect rotation angles of the pair of side detection parts.

5. The body type analysis device of claim 4,
wherein the center detection part and the pair of side detection parts are provided to be elastically rotatable around an arbitrary rotation shaft.

6. The body type analysis device of claim 5,
wherein the center encoder and the pair of side encoders are installed on the arbitrary rotation shaft.

7. The body type analysis device of claim 2,
wherein the detection unit is configured so that a separation distance between the pair of side detection parts is set to a distance capable of detecting a range of the shape of the spine diagnosed as a scoliosis patient with only one-time operation of the moving drive unit.

8. The body type analysis device of claim 1,
wherein the moving drive unit includes:
a moving bogie part configured to provide an installation location of the detection unit;
a moving rail part configured to provide a moving line to move the moving bogie part in a straight vertical direction; and
a moving drive part configured to provide a driving force to move the moving bogie part along the moving rail part.

9. The body type analysis device of claim 8,
wherein the moving rail part includes:
a screw rod arranged along the moving line, and having a male thread processed on an outer circumferential surface to be moved by an operation rotated by passing through a moving block provided on the moving bogie part; and
a rod support bracket configured to rotationally support both upper and lower ends of the screw rod.

10. The body type analysis device of claim 9,
wherein any one of the upper and lower ends of the screw rod is directly connected to a rotation shaft of the moving drive part.

11. The body type analysis device of claim 9,
wherein the moving rail part further includes: a sub rail provided to be spaced apart from the screw rod by a predetermined interval and parallel to the screw rod, and configured to support the moving of the moving bogie part.

12. The body type analysis device of claim 1,
wherein the moving drive unit is installed to be movable with respect to an analysis body part fixed to be vertically long.

13. The body type analysis device of claim 12,
wherein the analysis body part is provided with a fabric guide belt to be connected to the upper and lower ends of the moving drive unit and rotated in conjunction therewith.

14. The body type analysis device of claim 12,
wherein one side of the analysis body part is provided with a protective chain rail configured to protect an electric wire arranged to supply power to the moving drive unit, and
an electric wire protective chain moved to cover the electric wire arranged on the protect chain rail is coupled to the moving drive unit to be interlocked with the moving drive unit.
